# EUROPEAN PATENT APPLICATION

(11) **EP 2 832 361 A1**
(43) Date of publication of application: **04.02.2015**
(21) Application number: 13290175.2
(22) Date of filing: 29.07.2013
(51) Int. Cl.: A61K 38/09, A61K 9/00, A61K 47/02

(54) **Aqueous sustained release compositions of LHRH analogs**

(71) Applicant: IPSEN PHARMA S.A.S., 92100 Boulogne-Billancourt (FR)
(72) Inventor: The designation of the inventor has not yet been filed
(74) Representative: Retzler, Charlotte

(57) **Abstract**

The present invention relates to an aqueous pharmaceutical composition for a sustained release of a LHRH analog, in particular for a sustained release compatible with therapeutic treatments for at least 2 weeks. The pharmaceutical compositions are particularly useful for the treatment of diseases a LHRH analog is indicated for.

## Description

The present invention relates to an aqueous pharmaceutical composition for a sustained release of a LHRH analog, in particular for a sustained release compatible with therapeutic treatments of at least 2 weeks.

Luteinizing Hormone Releasing Hormone, known as LHRH or GnRH, is a decapeptide with the following formula: (pyro)Glu-His-Trp-Ser-Tyr-Gly-Leu-Arg-Pro-Gly-NH₂.

LHRH is released from the hypothalamus and binds to a receptor on the pituitary gland, causing the release of LH (Luteinizing Hormone) and FSH (Follicle - Stimulating Hormone). Subsequently, LH and FSH act on the gonads to stimulate the synthesis of steroid sex hormones. The pulsatile release of LHRH, and thereby the release of LH and FSH, controls the reproductive cycle in domestic animals and humans. Acute doses of LHRH agonists increase the levels of LH and steroid sex hormones in both animals and humans. Paradoxically, chronic doses of these agonists suppress the levels of LH and steroid hormones. Consequently, the effect of multiple doses of LHRH agonists is to suppress estrogen formation in the female and suppress testosterone formation in the male. The same effect is observed in both animals and humans after administration of acute or chronic doses of LHRH antagonists. LHRH agonists have been used or are under clinical investigation for the treatment of several hormone dependent diseases such as prostate cancer, prostatic hypertrophy, endometriosis, uterine fibroids, precocious puberty and breast cancer. They have also been used as contraceptives. For a review of LHRH analogs see J. Sandow, et al. in "Hypothalamic Hormones. Chemistry, Physiology, and Clinical Applications", edited by D. Gupta and W. Voeters, p. 307 (1978).

The treatments with LHRH analogs require continuous and/or repeated administration to the patient over an extended period of time. As repeated injections cause both inconvenience and discomfort to the patient, sustained release preparations are desirable.

In general, the release of the active ingredient is obtained by using biocompatible and/or biodegradable (co)polymer such as PLGA and the formulation is under the form of microimplant or microparticles in appropriate excipients.

The present invention relates to an aqueous sustained release formulation of LHRH analog, with a simple composition, in particular free of any non hydrosoluble, biocompatible and/or biodegradable (co)polymer or excipient, and which may be injected with classical needle such as insulin one.

The subject of the present invention is thus an aqueous pharmaceutical composition for a sustained release of an active ingredient for at least 2 weeks, comprising:
- as the active ingredient a LHRH analog,
- a salt, and
- water.

In the text herein below, unless otherwise indicated, the limits of a range of values are included in that range, especially in the expression "ranging from".

Unless otherwise indicated the following definitions are set forth to illustrate and define the meaning and scope of the various terms used to describe the invention herein.

The term "salt" as used herein refers to a monovalent or multivalent, organic or inorganic, anionic or cationic salt.

The term "buffer" as used herein refers to a solution containing an ionisable compound that resists changes in its pH and can then stabilize the pH of the composition in a specific range. A buffer generally consists in either a weak acid or its salts or a weak base and its salts, which is resistant to changes in pH.

The "injectability" which can be determined by measuring the injection force, refers to the suitability of the formulation for parenteral administration using a device for injection, like a syringe or an injector.

The term "stabilizer" as used herein means a pharmaceutically acceptable compound to prevent degradation, enhance the physical or chemical stability of the active substance (e.g. a compound having antioxidant properties or surfactants).

The term "surfactant" as used herein refers to a compound or excipient with surface active properties, used mainly in the present formulations to improve the aqueous solubility of the active ingredient, help to protect the active substance against degradation and limit active ingredient precipitation.

The term "antioxidant" as used herein refers to a compound having antioxidant properties in order to prevent oxidative degradation of the active ingredient and prevent oxidative degradation of the excipients.

The term "(co)polymer" means a polymer or copolymer or a mixture thereof.

The term "non hydrosoluble" is understood to mean non soluble in water. Preferably, a "non hydrosoluble" (co)polymer or excipient has solubility in water measured at 25° C less than 1 mg/mL, and preferably less than 0.1 mg/mL.

The term "biocompatible" means biologically compatible by not producing a toxic, injurious, or immunological response in living tissues, biological systems or biological functions.

The term "biodegradable" means capable of being decomposed by biological agents, biological (micro-)organisms, or when placed in biological fluids.

Unless otherwise stated, all percentages mentioned in the present invention are weight percentages.

The LHRH analog may be selected from triptorelin, deslorelin, nafarelin, histrelin, buserelin, goserelin, gonadorelin and leuprorelin or any pharmaceutically acceptable salt thereof. The active ingredient is in the form of a salt or as a free base.

In a particular embodiment, the LHRH analog is selected from triptorelin, deslorelin, nafarelin, histrelin, buserelin, goserelin, gonadorelin and leuprorelin or any pharmaceutically acceptable salt thereof.

Preferably the LHRH analog is triptorelin or a pharmaceutically acceptable salt thereof.

The salts of LHRH analog which can be used for the invention are preferably pharmaceutically acceptable salts of organic acids, such as those of acetic, phenylacetic, lactic, malic, pamoic, ascorbic, succinic, benzoic, methanesulphonic or toluenesulphonic acids, or pharmaceutically acceptable salts of inorganic acids, such as those of hydrochloric, hydrobromic, hydriodic, sulphuric or phosphoric acids.

According to one preferred embodiment, the LHRH analog is in a salt form.

Preferably the LHRH analog is in the form of an acetate or a phosphate salt, and more preferably from an acetate salt.

In a preferred embodiment of the invention, the LHRH analog is triptorelin and its pharmaceutical acceptable salt is acetate or phosphate, and preferably acetate.

According to another preferred embodiment, triptorelin is as a free base.

Whatever the form of the LHRH analog, i.e. salt form or free base, in the sense of the present invention, the amount of the LHRH analog, expressed for instance as a concentration or a percentage, refers to the LHRH analog as a free base.

Advantageously, the LHRH analog is present in a concentration ranging from 1 to 30 % by weight, preferably from 1 to 25 % by weight, and more preferably from 1.5 to 22 % by weight relative to the total weight of the composition.

Preferably the LHRH analog is triptorelin and is present in a concentration ranging from 1 to 30 % by weight, preferably from 1 to 25 % by weight, and more preferably de 1.5 to 22 % by weight relative to the total weight of the composition.

A composition according to the present invention comprises a salt. The salt may be selected from phosphate, sulphate, citrate, acetate, succinate, carbonate or chloride salts.

When the salt is a sulphate salt, it may be selected from sodium sulphate or ammonium sulphate.

In a preferred embodiment, the salt is chosen from a phosphate, sulphate, citrate, acetate, succinate, carbonate or chloride salts.

Preferably the salt is chosen from a phosphate salt or a sulphate salt.

In one embodiment of the invention, the salt is a sulphate salt.

In another preferred embodiment, the salt is a sulphate salt and it is selected from sodium sulphate and ammonium sulphate. Preferably the sulphate salt is sodium sulphate.

In another embodiment of the invention, the salt is a phosphate salt.

In the particular case of the phosphate salt, the phosphate salt may be a phosphate buffer. Phosphate buffer can be prepared by dissolving sodium dihydrogene phosphate dihydrate in water and adjusting pH to 7.5 with sodium hydroxide.

Preferably the phosphate salt is a phosphate buffer.

In a preferred embodiment, the present invention is an aqueous pharmaceutical composition for a sustained release of an active ingredient for at least 2 weeks, comprising:
- triptorelin as the active ingredient,
- a salt, and
- water, triptorelin being present in a concentration ranging from 1 to 30 % by weight relative to the total weight of the composition.

Preferably triptorelin is present in a concentration ranging from 1 to 25 % by weight, and more preferably from 1.5 to 22 % by weight relative to the total weight of the composition.

In a particular preferred embodiment, the pH of the final composition is ranging from 4 to 9.

In another embodiment, the salt is a sulphate salt and preferably ammonium sulphate. Preferably, the salt is ammonium sulphate and the pH of ammonium sulphate solution is ranging from 4 to 9, and more preferably from 5 to 8.

In a preferred embodiment, the salt is a sulphate salt and preferably sodium sulphate. Preferably, the salt is sodium sulphate and the pH of sodium sulphate solution is ranging from 4 to 9, and more preferably from 4 to 7.

In another preferred embodiment, the salt is a phosphate salt. Preferably, the salt is a phosphate salt and the pH of the phosphate salt solution is ranging from 4 to 9, and more preferably from 6 to 9.

In a preferred embodiment of the invention, the molar ratio of salt to LHRH analog ranges from approximately 0.1:1 (0.1 ± 0.01) to approximately 2:1 (2 ± 0.2).

In a more preferred embodiment of the invention, the active ingredient is triptorelin or a pharmaceutically acceptable salt thereof, the salt is a phosphate salt and the molar ratio of phosphate to triptorelin ranges from approximately 0.1:1 (0.1 ± 0.01) to approximately 2:1 (2 ± 0.2).

Preferably the molar ratio of phosphate to triptorelin ranges from approximately 0.15:1 (0.15 ± 0.015) to approximately 1.5:1 (1.5 ± 0.15).

One subject of the present invention is an aqueous pharmaceutical composition for a sustained release of an active ingredient for at least 2 weeks, comprising:
- from 1 to 30 % (w/w) of triptorelin as the active ingredient,
- a phosphate salt, and
- water,
the molar ratio of phosphate to triptorelin ranging from approximately 0.1:1 (0.1 ± 0.01) to approximately 2:1 (2 ± 0.2), preferably from approximately 0.15:1 (0.15 ± 0.015) to approximately 1.5:1 (1.5 ± 0.15).

In another more preferred embodiment of the invention, the active ingredient is triptorelin or a pharmaceutically acceptable salt thereof, the salt is sodium sulphate and the molar ratio of sulphate to triptorelin ranges from approximately 0.1:1 (0.1 ± 0.01) to approximately 2:1 (2 ± 0.2).

Preferably the molar ratio of sulphate to triptorelin ranges from approximately 0.15:1 (0.15 ± 0.015) to approximately 1.5:1 (1.5 ± 0.15).

Another subject of the present invention is an aqueous pharmaceutical composition for a sustained release of an active ingredient for at least 2 weeks, comprising:
- from 1 to 30 % (w/w) of triptorelin as the active ingredient,
- sodium sulphate, and
- water,
the molar ratio of sulphate to triptorelin ranging from approximately 0.1:1 (0.1 ± 0.01) to approximately 2:1 (2 ± 0.2), preferably from approximately 0.15:1 (0.15 ± 0.015) to approximately 1.5:1 (1.5 ± 0.15).

In another more preferred embodiment of the invention, the active ingredient is triptorelin or a pharmaceutically acceptable salt thereof, the salt is ammonium sulphate and the molar ratio of sulphate to triptorelin ranges from approximately 0.1:1 (0.1 ± 0.01) to approximately 2:1 (2 ± 0.2).

Preferably the molar ratio of sulphate to triptorelin ranges from approximately 0.15:1 (0.15 ± 0.015) to approximately 1.5:1 (1.5 ± 0.15).

Another subject of the present invention is an aqueous pharmaceutical composition for a sustained release of an active ingredient for at least 2 weeks, comprising:
- from 1 to 30 % (w/w) of triptorelin as the active ingredient,
- ammonium sulphate, and
- water,
the molar ratio of sulphate to triptorelin ranging from approximately 0.1:1 (0.1 ± 0.01) to approximately 2:1 (2 ± 0.2), preferably from approximately 0.15:1 (0.15 ± 0.015) to approximately 1.5:1 (1.5 ± 0.15).

In another preferred embodiment, the aqueous pharmaceutical composition according to the present invention is free of any non hydrosoluble, biocompatible and/or biodegradable, (co)polymer or excipient or a mixture thereof. This means that the content of any non hydrosoluble, biocompatible and/or biodegradable, (co)polymer or excipient in the composition is less than 0.1 % by weight (w/w).

A classical and well-described way to provide a pharmaceutical composition with a sustained release of an active pharmaceutical ingredient after administration is the use of biocompatible (co)polymers such as polylactides (PLA), polyglycolides (PLG), poly lactide-co-glycolides (PLGA), polyalkylcyanoacrylates, poly-ε-caprolactones and any (co)polymer agents obtained by combination or modification of these biocompatible (co)polymers. Such (co)polymers which are not hydrosoluble form the biodegradable matrix of microparticles or solid implants, which is progressively eroded when administered into the body.

In another preferred embodiment, the aqueous pharmaceutical composition of the present invention is for a sustained release of an active ingredient for at least 2 weeks, free of any non hydrosoluble, biocompatible and/biodegradable, (co)polymer, and comprising:
- triptorelin as the active ingredient,
- a salt, and
- water,
triptorelin being present in a concentration ranging from 1 to 30 % by weight relative to the total weight of the composition.

Preferably triptorelin is present in a concentration ranging from 1 to 25% by weight, and more preferably from 1.5 to 22 % by weight relative to the total weight of the composition.

In a particular embodiment, an aqueous pharmaceutical composition of the present invention is free of any non hydrosoluble, biocompatible and/or biodegradable, (co)polymer, and comprises:
- from 1 to 30 % (w/w) of triptorelin as the active ingredient,
- a phosphate salt, and
- water
the molar ratio of phosphate to triptorelin ranging from approximately 0.1:1 (0.1 ± 0.01) to approximately 2:1 (2 ± 0.2).

Preferably the molar ratio of phosphate to triptorelin ranges from approximately 0.15:1 (0.15 ± 0.015) to approximately 1.5:1 (1.5 ± 0.15).

In another particular embodiment, an aqueous pharmaceutical composition of the present invention is free of any non hydrosoluble, biocompatible and/or biodegradable, (co)polymer, and comprises:
- from 1 to 30 % (w/w) of triptorelin as the active ingredient,
- sodium sulphate, and
- water
the molar ratio of sulphate to triptorelin ranging from approximately 0.1:1 (0.1 ± 0.01) to approximately 2:1 (2 ± 0.2).

Preferably the molar ratio of sulphate to triptorelin ranges from approximately 0.15:1 (0.15 ± 0.015) to approximately 1.5:1 (1.5 ± 0.15).

In another particular embodiment, an aqueous pharmaceutical composition of the present invention is free of any non hydrosoluble, biocompatible and/or biodegradable, (co)polymer, and comprises:
- from 1 to 30 % (w/w) of triptorelin as the active ingredient,
- ammonium sulphate, and
- water
the molar ratio of sulphate to triptorelin ranging from approximately 0.1:1 (0.1 ± 0.01) to approximately 2:1 (2 ± 0.2).

Preferably the molar ratio of sulphate to triptorelin ranges from approximately 0.15:1 (0.15 ± 0.015) to approximately 1.5:1 (1.5 ± 0.15).

A composition according to the present invention may also contain other hydrosoluble additives usually used in such pharmaceutical compositions such as, for instance, stabilizers, antioxidants or surfactants.

Stabilizers or surfactants may be selected from metal ions, fatty acids and salts thereof, fatty alcohols, polyoxyethers, poloxamers, polyols such as trehalose, mannitol, saccharose and dextrose, polysorbates and polyoxyethylene fatty acid esters.

A composition according to the present invention may contain one or more multivalent metal ions as stabilizer.

In a particular embodiment, the composition of the present invention comprises one or more multivalent metal ions, and preferably one or more divalent metal cations.

In a preferred embodiment, the multivalent metal ion is chosen from Zn²⁺, Cu²⁺, Mg²⁺, Fe²⁺ and Ca²⁺.

Antioxidants may be selected from amino acids such as methionine, histidine, tryptophan and glutathione, chelating agents such as disodium edetate and citric acid, ascorbic acid, sodium metabisulfite, monothioglycerol.

If present, the amount in weight (w/w) of these additives is lower than 5.0 % of the pharmaceutical composition, and preferably lower than 1.0 %.

A composition according to the present invention may also contain a pH buffer.

The composition according to the present invention may be prepared by mixing the active ingredient, the salt and water. The active ingredient may be pre-solubilized in water, then the salt is added as a solution in water for injection (WFI). In case other additives are used, they are dissolved either in the peptide solution or in the salt solution before their mixing.

The pharmaceutical composition according to the present invention is a ready-to-use composition, sealed in a syringe type device.

A pharmaceutical composition according to the present invention is administered by the parenteral route. In a preferred embodiment, the composition of the present invention is administered by subcutaneous, intramuscular or deep-subcutaneous injection, and more preferably subcutaneous injection.

A pharmaceutical composition according to the present invention allows a sustained release of triptorelin in humans for at least 2 weeks. Such release is obtained without any (co)polymeric matrix in the composition, in particular without any non hydrosoluble, biocompatible and/ biodegradable, (co)polymeric agents usually used in sustained release composition such as polylactides (PLA), polyglycolides (PLG), poly lactide-co-glycolides (PLGA), polyalkylcyanoacrylates, poly-ε-caprolactones and any (co)polymer agents obtained by combination or modification of these biocompatible (co)polymers.

In a preferred embodiment, the sustained release of LHRH analog is of at least 2 to 6 weeks in humans.

In a preferred embodiment, the sustained release of LHRH analog is of at least 1 month in humans.

The composition according to the invention allows a sustained release for at least 14 days, 21 days, 28 days, 35 days or 42 days.

In a preferred embodiment, the composition according to the invention allows a sustained release for at least 14 days. In another preferred embodiment, the composition according to the invention allows a sustained release for at least 21 days. In another preferred embodiment, the composition according to the invention allows a sustained release for at least 28 days. In a more preferred embodiment, the composition according to the invention allows a sustained release for at least 35 days. In another more preferred embodiment, the composition of the present invention allows a sustained release for at least 42 days.

The LHRH agonist and antagonist compounds of the invention are useful for treatment of precocious puberty, prostate cancer, prostatic hypertrophy, endometriosis, uterine fibroids, breast cancer, acne, premenstrual syndrome, polycystic ovary syndrome and diseases which result from excessive gonadal hormone production in either sex. LHRH agonists and antagonists are also useful for controlling reproduction in both females and males. LHRH agonists, when administered in pulses, are useful as fertility promoters. The LHRH agonist compounds of the invention may also be useful for growth promotion in female animals and for spawning promotion in fish.

A pharmaceutical composition according to the present invention may be useful in the treatment of precocious puberty, prostate cancer, prostatic hypertrophy, endometriosis, uterine fibroids, breast cancer, acne, premenstrual syndrome, polycystic ovary syndrome and diseases which result from excessive gonadal hormone production in either sex. In a prefered embodiment, a pharmaceutical composition according to the present invention is useful in the treatment of prostate cancer.

Unless otherwise indicated, all technical and scientific terms used herein have the same meaning as commonly understood by a specialist in the domain associated with this invention.

The following examples are presented to illustrate the above procedures and should not be considered as limiting the scope of the invention.

### Experimental part

### Example 1: Preparation process

Various compositions according to the present invention are prepared with a LHRH analog as active ingredient according to the following process:

### 1. Preparation of media

The different media used to manufacture the prototypes were prepared as follows:

### 1.1. Phosphate buffer 200 mM pH 7.5

1.560 g of NaH₂PO₄ 2H₂O are weighed in a 50 mL flask and dissolved in 45 mL of water for injection under magnetic stirring. Then the pH is adjusted to a value of about 7.5 with NaOH 10 N. The solution is then completed to 50 mL with WFI and the pH is measured.

### 1.2.Solution of sodium sulphate at 200 mM

1.420 g of Na₂SO₄ are weighed in a 50 mL flask and dissolved in WFI under magnetic stirring. The solution is then completed to 50 mL.

### 1.3. Solution of ammonium sulphate at 200 mM

1.321 g of (NH₄)₂SO₄ are weighed in a 50 mL flask and dissolved in WFI under magnetic stirring. The solution is then completed to 50 mL.

### 2. Manufacture of prototypes

A manual push-pull syringe device is used to ensure the homogenization of the preparation.

The peptide is weighed in the first syringe. Either 115.8 mg or 34.7 mg of peptide are introduced in the first syringe to obtain a final triptorelin concentration of respectively 10% w/w or 3% w/w.

This first syringe containing the peptide is connected to a second syringe containing 500 µL of water for injection. The peptide solubilisation in water is obtained by ensuring a minimum of five transfers. Then, the empty syringe is taken off and filled with 500 µL of one of the media. After connection with the syringe containing the peptide solution, the homogenization with the medium is obtained by ensuring a minimum of ten transfers.

Prototypes are stored at room temperature protected from light.

A minimum of ten transfers is done 1 h, 24 h and 48 h after the preparation.

Examples of formulation compositions are reported in Table 1:

**Table 1**

| Composition # | LHRH analog salt | Content of LHRH analog | Salt | Salt / triptorelin molar ratio | pH of final composition |
|---|---|---|---|---|---|
| 1 | Triptorelin acetate (1) | 10% | Phosphate | 1.4 | ND |
| 2 | Triptorelin acetate (1) | 3% | Sodium sulphate | 1.4 | 5.9 |
| 3 | Triptorelin acetate (1) | 10% | Ammonium sulphate | 1.4 | 5.9 |
| 4 | Triptorelin acetate (1) | 3 % (2) | Phosphate | 1.4 | ND |

| | | | | | |
|---|---|---|---|---|---|
| (1) pure peptide content in the raw material: 86.4 % (2) dilution of composition 1 in phosphate buffer 100 mM ND: not determined | | | | | |

The content of LHRH analog is expressed in weight percentage of product relative to the total weight of the composition.

Composition 4 corresponds to a diluted composition 1.

### 3. Manufacture of other prototypes

### 3.1. Manufacture of a 20 % w/w prototype at a phosphate/peptide ratio of 0.6

1,744 g of triptorelin acetate are weighed in a 25 mL glass bottle and dissolved in 5.625 mL of phosphate buffer under magnetic stirring. Additional 1.875 mL of phosphate buffer are added slowly under magnetic stirring to ensure adequate homogenization of the prototype. The pH of the final composition is 5.7. The formulation, a white and dense semi-solid, is then filled in 1.2 mL plastic syringes fitted with a 18G needle. This composition according to the present invention presents a maximum injection force defined by a SIF of 12 N at a 200 mm/min speed.

Phosphate buffer is prepared by dissolving 1.43 g of sodium dihydrogene phosphate dihydrate in 80 mL of WFI, adjusting pH to 7.5 with sodium hydroxide 5 N and adjusting final volume to 100 mL.

### 3.2. Manufacture of a 20 % w/w prototype at a sulphate/peptide ratio of 0.6

1,744 g of triptorelin acetate are weighed in a 25 mL glass bottle and dissolved in 5.625 mL of sodium sulphate solution under magnetic stirring. Additional 1.875 mL of sodium sulphate solution are added slowly under magnetic stirring to ensure adequate homogenization of the prototype. The pH of the final composition is 5.4. A white and dense semi-solid is obtained.

### Sodium sulphate solution is prepared by dissolving 1.30 g of sodium sulphate in 100 mL of WFI.

### 3.3. Manufacture of a 10 % w/w prototype at a phosphate/peptide ratio of 0.2

0.873 g of triptorelin acetate are weighed in a 10 mL beaker and dissolved in 3.75 mL of WFI under magnetic stirring. 3.75 mL of phosphate buffer are added slowly under magnetic stirring to ensure adequate homogenization of the prototype. A white semi-solid is obtained.

Phosphate buffer is prepared by dissolving 0.536 g of sodium dihydrogene phosphate dehydrate in 80 mL of WFI, adjusting pH to 7.5 with sodium hydroxide 5 N and adjusting final volume to 100 mL.

### 3.4. Manufacture of a 10 % w/w prototype at a sulphate/peptide ratio of 0.2

0.873 g of triptorelin acetate are weighed in a 25 mL glass bottle and dissolved in 3.75 mL of WFI under magnetic stirring. 3.75 mL of a sodium sulphate solution are added slowly under magnetic stirring to ensure adequate homogenization of the prototype. The pH of the final composition is 5.2. A creamy and viscous semi-solid is obtained.

### Sodium sulphate solution is prepared by dissolving 0.2175 g of sodium sulphate in 50 mL of WFI.

### Example 2: Stability study

Prototypes were stored at room temperature shielded from the light and subsequently analysed by HPLC at various time points before the *in vivo* testing.

The stability data are shown in Tables 2 and 3.

**Table 2**

| Composition # | Peptide content | Medium | HPLC analysis | | | |
|---|---|---|---|---|---|---|
| | | | Content (mg/mL) | | Sum of impurities (%) > 0.1% | |
| | | | D13 | D38 | D13 | D38 |
| 1 | 10% | Phosphate salt | 91.0 | 92.3 | 0.26 | 0.31 |
| 2 | 3% | Sodium sulphate | 23.8 | 25.8 | 0.20 | 0.20 |
| 3 | 10% | Ammonium sulphate | 88.5 | 98.2 | 0.20 | 0.21 |

The amount of impurities does not increase for 38 days at room temperature.

**Table 3**

| Composition # | Peptide content | Medium | HPLC analysis | |
|---|---|---|---|---|
| | | | Content (mg/mL) | Sum of impurities (%) > 0.1 % |
| | | | D8 after dilution | D8 after dilution |
| 4 | 3% (2) | Phosphate salt | 34.9 | 0.20 |

### Example 3: Pharmacokinetic (PK) studies

The pharmaceutical compositions identified in Table 1 are injected in rat at different doses of triptorelin salt.

The single intramuscular administered doses are reported in Table 4:

**Table 4**

| | | | | |
|---|---|---|---|---|
| Composition # | 1 | 2 | 3 | 4 |
| Doses (µg/kg) | 2475 | 825 | 2475 | 825 |

A total of 6 male Wistar rats per formulation are used at the beginning of the study.

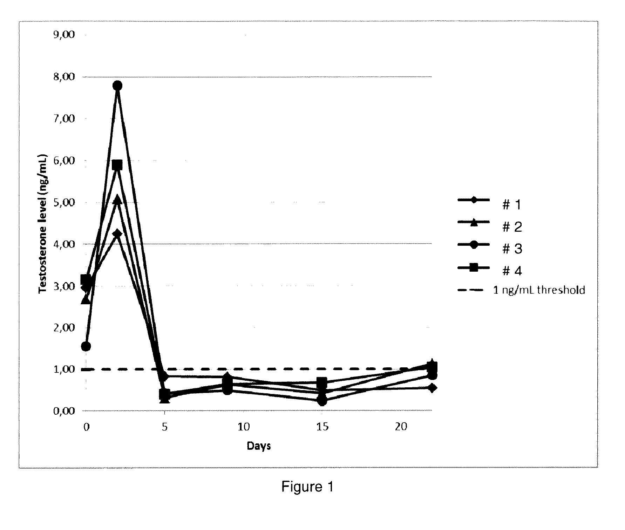

Blood samples are obtained from unanesthetized animals by direct venipuncture from the jugular vein, before injection (time 0), 2, 5, 9, 15 and 22 days after administration. Testosterone levels are determined in rats for each composition and resulting mean are presented in Figure 1.

Results are compared to the threshold of 1 ng/mL below which a chemical castration is efficient.

All compositions have testosterone concentrations below the 1 ng/mL threshold from 5 to 15 days after single intramuscular administration of the composition of the invention. This pharmacokinetic study demonstrates the efficacy of these compositions.

## Claims

1. Aqueous pharmaceutical composition for a sustained release of an active ingredient for at least 2 weeks, comprising:
- as the active ingredient a LHRH analog,
- a salt, and
- water.

2. Composition according to claim 1, wherein the LHRH analog is selected from triptorelin, deslorelin, nafarelin, histrelin, buserelin, goserelin, gonadorelin and leuprorelin or any pharmaceutically acceptable salt thereof.

3. Composition according to any one of the preceding claims, wherein the LHRH analog is triptorelin or a pharmaceutically acceptable salt thereof.

4. Composition according to any one of the preceding claims, wherein the LHRH analog is in a salt form.

5. Composition according to any one of the preceding claims, wherein the LHRH analog is triptorelin and its pharmaceutically acceptable salt is acetate or phosphate, and preferably acetate.

6. Composition according to any one of the preceding claims, wherein the LHRH analog is present in a concentration ranging from 1 to 30 % by weight, preferably from 1 to 25 % by weight, and more preferably from 1.5 to 22 % by weight relative to the total weight of the composition.

7. Composition according to any one of the preceding claims, wherein the salt is chosen from a phosphate, sulphate, citrate, acetate, succinate, carbonate or chloride salts, and more preferably from phosphate salt or sulphate salt.

8. Composition according to claim 7, wherein the sulphate salt is sodium sulphate or ammonium sulphate, and preferably is sodium sulphate.

9. Composition according to any of the preceding claims, wherein the pH of the final composition is ranging from 4 to 9.

10. Composition according to any of the preceding claims, wherein the molar ratio of salt to LHRH analog ranges from approximately 0.1:1 to approximately 2:1.

11. Composition according to claim 7, wherein the active ingredient is triptorelin or a pharmaceutically acceptable salt thereof, the salt is a phosphate salt and the molar ratio of phosphate to triptorelin ranges from approximately 0.1:1 to approximately 2:1, preferably from approximately 0.15:1 to approximately 1.5:1.

12. Composition according to claim 8, wherein the active ingredient is triptorelin or a pharmaceutically acceptable salt thereof, the salt is sodium sulphate and the molar ratio of sulphate to triptorelin ranges from approximately 0.1:1 to approximately 2:1, preferably from approximately 0.15:1 to approximately 1.5:1.

13. Composition according to claim 8, wherein the active ingredient is triptorelin or a pharmaceutically acceptable salt thereof, the salt is ammonium sulphate and the molar ratio of sulphate to triptorelin ranges from approximately 0.1:1 to approximately 2:1, preferably from approximately 0.15:1 to approximately 1.5:1.

14. Composition according to any one of the preceding claims, which is free of any non hydrosoluble, biocompatible and/or biodegradable, (co)polymer or excipient.

15. Composition according to any one of the preceding claims, further comprising one or more multivalent metal ions, preferably one or more divalent metal cations.

16. Composition according to claim 15, wherein the multivalent metal ion is chosen from Zn²⁺, Cu²⁺, Mg²⁺, Fe²⁺ and Ca²⁺.

17. Composition according to any one of the preceding claims, which is a ready-to-use composition, sealed in a syringe type device.
